(19) Europäisches Patentamt
European Patent Office
Office européen des brevets

(11) Veröffentlichungsnummer : **0 099 981**
**B1**

(12) # EUROPÄISCHE PATENTSCHRIFT

(45) Veröffentlichungstag der Patentschrift : **02.01.86**

(51) Int. Cl.⁴ : **C 07 C 59/153**, C 07 C 69/67

(21) Anmeldenummer : **83105712.0**

(22) Anmeldetag : **10.06.83**

(54) **Verfahren zur Herstellung von Glyoxylsäure und Glyoxylsäurederivaten.**

(30) Priorität : **02.07.82 DE 3224795**

(43) Veröffentlichungstag der Anmeldung : **08.02.84 Patentblatt 84/06**

(45) Bekanntmachung des Hinweises auf die Patenterteilung : **02.01.86 Patentblatt 86/01**

(84) Benannte Vertragsstaaten : **AT BE CH DE FR GB IT LI LU NL SE**

(56) Entgegenhaltungen :
**FR-A- 2 383 911**
**US-A- 3 637 721**
**US-A- 3 644 508**
**US-A- 3 705 922**

(73) Patentinhaber : **CHEMIE LINZ AKTIENGESELLSCHAFT**
**St. Peter-Strasse 25**
**A-4020 Linz (AT)**
**BE CH FR GB IT LI LU NL SE AT**
**Lentia Gesellschaft mit beschränkter Haftung**
**Arabellastrasse 4 Postfach 81 05 08**
**D-8000 München 81 (DE)**
**DE**

(72) Erfinder : **Sajtos, Alexander, Dipl. Ing.**
**Weissenwolffstrasse 6**
**A-4020 Linz (AT)**
Erfinder : **Wechsberg, Manfred, Dr.**
**Im Blumengrund 9/35**
**A-4020 Linz (AT)**
Erfinder : **Roithner, Erich**
**Stülzgasse 14**
**A-4020 Linz (AT)**

**EP 0 099 981 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Glyoxylsäurealkylesterhalbacetalen oder Glyoxylsäure durch Ozonolyse eines Maleinsäurealkylesters und anschließende katalytische Hydrierung der Ozonolyseprodukte.

Die Herstellung von Carbonylverbindungen aus ungesättigten organischen Kohlenstoffverbindungen mit einer oder mehreren olefinischen Doppelbindungen im Molekül mittels eines Ozonolyse- und Reduktionsprozesses ist bekannt. Bei Anwendung dieser Methode bereitet die Reduktion immer wieder Schwierigkeiten, da die peroxidaltigen Ozonolyseprodukte instabil sind und in Anwesenheit von metallischen Hydrierkatalysatoren besonders leicht Umlagerungen erfahren, bevor sie zu den entsprechenden Carbonylverbindungen reduziert werden können. Außerdem werden bei Edelmetallkatalysatoren im Kontakt mit peroxidhaltigen Lösungen Aktivitätsverluste des Katalysators beobachtet. Dadurch entstehen erhebliche Ausbeuteverluste und Schwierigkeiten bei der Reindarstellung der Endprodukte.

Zur Vermeidung dieser Schwierigkeiten wird in der US-PS-3,145232 ein Verfahren empfohlen, bei dem die Reduktion unmittelbar nach der Ozonolyse bei Temperaturen unterhalb von − 40 °C in Anwesenheit von äquivalenten Mengen eines Trialkylphosphits durchgeführt wird. Neben dem apparativen Aufwand zur Herstellung der extrem tiefen Reaktionstemperaturen erfordert eine solche Reaktionsführung die Verwendung von absolut wasserfreien Lösungsmitteln, da die Trialkylphosphite in wasserhältigen Lösungsmitteln äußerst rasch hydrolysiert werden. Außerdem bereitet die Abtrennung der freien Carbonylverbindungen von den bei der Reduktion entstehenden Phosphatestern erhebliche Schwierigkeiten.

Da festgestellt wurde, daß sich tiefe Reaktionstemperaturen nachteilig auf die Aktivität der eingesetzten Reduktionsmittel auswirken und deshalb Ausbeuteverluste entstehen, wird nach einem Verfahren, wie es in der US-PS-3,637721 beschrieben ist, zwar die Ozonolyse der olefinischen Doppelbindung bei Temperaturen von − 50 °C durchgeführt, während die Reaktionstemperaturen im Verlaufe der Reduktion mit aliphatischen oder aromatischen Disulfiden bis auf 50 °C gesteigert wird. Für die Herstellung der Glyoxylsäure durch Ozonolyse von Maleinsäure und Reduktion mit Dimethylsulfid ist eine 91 %ige Ausbeute angegeben, die allerdings durch Bildung des unlöslichen 2,4-Dinitrophenylhydrazinderivats der Glyoxylsäure bestimmt wird, da die Abtrennung der freien Glyoxylsäure vom entstandenen Dimethylsulfoxid undurchführbar ist. An dieser Stelle ist auch die Herstellung von Glyoxylsäurealkylesterhalbacetalen aus Maleinsäureäthyl- oder Maleinsäuremethylester beschrieben, wobei aber die Abtrennung der Glyoxylsäurealkylesterhalbacetale von Dimethylsulfoxid nach der Reduktion ebenfalls nur unvollständig gelingt.

In der US-PS-3,705.922 ist daher ein verbessertes Verfahren zur Herstellung von Glyoxylsäurehalbacetalen beschrieben, bei dem Maleinsäure mit einem Überschuß an Ozon umgesetzt wird und die peroxidhaltigen Ozonolyseprodukte durch katalytische Hydrierung in Anwesenheit von Palladium auf einem Aluminiumoxidträger reduziert werden.

Während im zuletzt genannten Verfahren zwar auf tiefe Temperaturen und den Einsatz von teuren, toxischen und übelriechenden Reduktionsmitteln, die sich nach der Umwandlung in die oxidierte Form überhaupt nicht oder nur schwer aus dem Reaktionsgemisch entfernen lassen, verzichtet werden kann, wird andererseits der Verbrauch eines sehr teuren und spezifischen Katalysatormaterials in Kauf genommen. Da Edelmetallkatalysatoren bei längerem Kontakt mit organischen Peroxiden desaktiviert werden, hängt hier die Ausbeute bei der Hydrierung von der Menge und der Zusammensetzung des Hydrierkatalysators ab. Wie aus einem Vergleich der Beispiele in der US-PS-3,705.922 hervorgeht, nimmt die Ausbeute trotz entsprechend verlängerter Reaktionszeit um etwa 10 % ab, wenn bei gleicher Ansatzgröße anstelle von 0,5 nur 0,2 g des $Pd/Al_2O_3$ Katalysators verwendet werden. Völlig unwirtschaftlich wird das Verfahren dann, wenn anstelle eines Palladiumkatalysators mit Aluminiumoxid als Trägermaterial ein gebräuchlicher Katalysator wie Pd/Kohle verwendet wird. In der US-PS-3,705.922 sind auch keine Angaben über die Regenerierung und Wiederverwendbarkeit des verbrauchten Katalysatormaterials enthalten. Die den bekannten Verfahren anhaftenden Nachteile können gemäß der vorliegenden Erfindung überraschenderweise durch ein Verfahren vermieden werden, bei dem man zur ozonolytischen Spaltung der olefinischen Doppelbindung ein Moläquivalent Ozon einsetzt und die peroxidhältigen Ozonolyseprodukte in verdünnter alkoholischer Lösung bei einer sehr niedrigen Konzentration an Peroxiden durch katalytische Hydrierung sehr rasch reduziert.

Gegenstand der Erfindung ist demnach ein Verfahren zur Herstellung von Glyoxylsäurealkylesterhalbacetalen mit der allgemeinen Formel

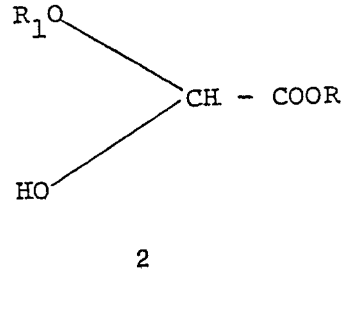

(I)

in der R einen Alkylrest mit 1 bis 10 C-Atomen und $R_1$ einen Alkylrest mit 1 bis 4 C-Atomen bedeuten, durch Ozonolyse eines Maleinsäurederivats, gefolgt von katalytischer Hydrierung der Ozonolyse-produkte, dadurch gekennzeichnet, daß man

a) einen Maleinsäuredialkylester mit 1 bis 10 C-Atomen im Alkylrest in einem aliphatischen Alkohol der Formel $R_1OH$, wobei $R_1$ die oben angegebene Bedeutung hat, löst und bei Temperaturen von − 80 bis + 20 °C mit der äquivalenten Menge Ozon umsetzt, worauf man

b) die dabei entstehende peroxidhältige Lösung in eine Suspension des Hydrierkatalysators in dem in Stufe a) bei der Ozonolyse verwendeten Alkohol der Formel $R_1OH$, mit $R_1$ mit der oben angegebenen Bedeutung, kontinuierlich in einer solchen Dosierung einspeist, daß über den ganzen Verlauf der Hydrierung im Hydrierreaktor ein Peroxidgehalt von maximal 0,1 Mol/l eingestellt und aufrechterhalten wird, und bei pH 2 bis pH 7 bei Temperaturen von 15 °C bis 45 °C unter einem Druck von 1 bis 20 bar hydriert.

Ein weiterer Gegenstand der Erfindung ist ein Verfahren zur Herstellung von Glyoxylsäure, welches dadurch gekennzeichnet ist, daß ein nach dem erfindungsgemäßen Verfahren hergestelltes Glyoxylsäurealkylesterhalbacetal der allgemeinen Formel I verseift wird.

Als Ausgangsmaterialien für das erfindungsgemäße Verfahren werden wegen der leichten Zugänglichkeit vorzugsweise Maleinsäuredimethyl- und Maleinsäurediäthylester verwendet. Ebenso geeignet sind aber auch die höheren Alkylester, beispielsweise der Dibutyl- oder Dioctylester. Es ist auch möglich, Maleinsäureanhydrid in Gegenwart eines niedrigen aliphatischen Alkohols unter saurer Katalyse, beispielsweise durch Zugabe eines stark sauren Ionenaustauschers in der H-Form oder katalytischer Mengen einer Mineralsäure, zu verestern und die dabei gebildete Lösung des entsprechenden Maleinsäuredialkylesters direkt der Ozonolyse zu unterwerfen. Dabei ist auf eine möglichst vollständige Veresterung des Maleinsäureanhydrids zu achten.

Die Ozonolyse wird vorzugsweise bei Temperaturen von − 20 °C bis + 10 °C durchgeführt, wobei die Einhaltung einer Temperatur im Bereich von − 10 bis + 5 °C wiederum besonders bevorzugt ist. Beim erfindungsgemäßen Verfahren wird der Maleinsäuredialkylester mit der äquivalenten Menge an Ozon umgesetzt, wobei unter den angegebenen Verfahrensbedingungen Ozon quantitativ aufgenommen wird und stöchiometrische Mengen des jeweils eingesetzten Maleinsäuredialkylesters verbraucht werden. Es sind daher nach Beendigung der Ozonisierung keine Maßnahmen erforderlich, um überschüssiges oder nicht umgesetztes Ozon vor der Hydrierung aus dem Reaktionsgemisch zu vertreiben.

Dem bei der Ozonolyse und der Hydrierung als Lösungsmittel verwendeten niedrigen aliphatischen Alkohol kommt insoferne Bedeutung zu, als dieser Alkohol an der Acetalbildung des Glyoxylsäurealkylesterhalbacetals der Formel I teilnimmt. Die Reaktionsfolge kann schematisch durch folgende Formelübersicht dargestellt werden, wobei R und $R_1$ die gleiche Bedeutung wie in Formel I besitzen :

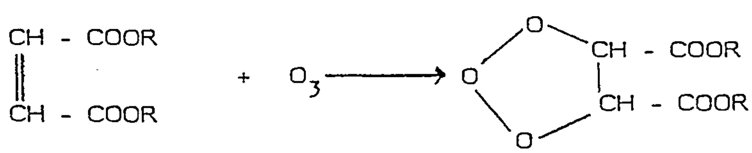

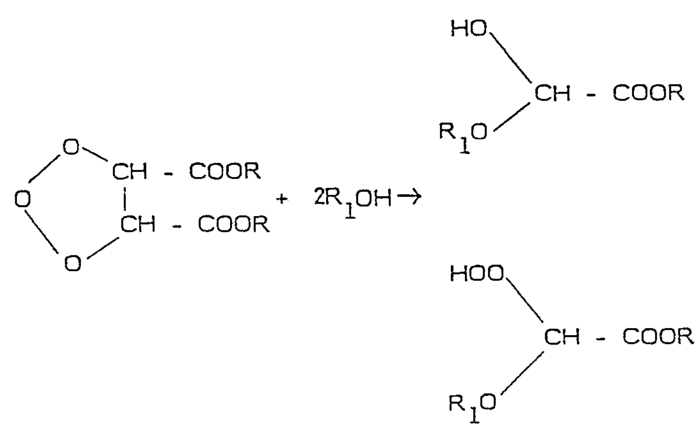

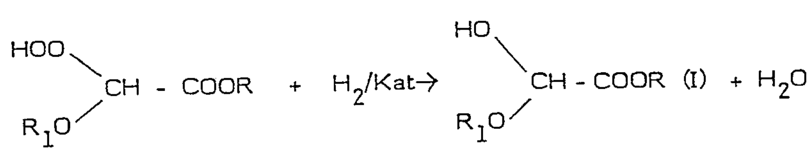

Für die Durchführung der Ozonolyse und Hydrierung kommen als alkoholische Lösungsmittel der Formel $R_1OH$ vor allem Methanol oder Äthanol in Betracht, wobei wiederum die Verwendung von Methanol besonders bevorzugt ist.

Die katalytische Hydrierung der Ozonolyseprodukte wird beim erfindungsgemäßen Verfahren in stark verdünnter alkoholischer Lösung durchgeführt, wobei durch die im folgenden beschriebene Maßnahme dafür Sorge getragen wird, daß während der gesamten Hydrierung ein Peroxidgehalt von höchstens 0,1 Mol/l, vorzugsweise von höchstens 0,05 Mol/l, und insbesondere von höchstens 0,02 Mol/l eingestellt und aufrecht erhalten wird. Dazu wird in einem Hydrierreaktor eine Suspension des Katalysators in dem bei der Ozonolyse als Lösungsmittel verwendeten Alkohol der Formel $R_1OH$ vorgelegt und die bei der Ozonisierung erhaltene Lösung mittels einer regelbaren Dosiervorrichtung kontinuierlich eingespeist. Bei der Zugabe der Ozonolyselösung zu Beginn und im Verlaufe der Hydrierung ist selbstverständlich darauf zu achten, daß durch die zugeführte Menge der peroxidhältigen Ozonolyseprodukte der oben angegebene Peroxidgehalt im Hydrierreaktor nicht überschritten wird.

Durch die geringe Konzentration an peroxidhältiger Ozonolyselösung im Reaktionsmedium während des eingetlichen Hydriervorganges infolge der kontinuierlichen Einspeisung ist eine rasche Reduktion gewährleistet. Auf diese Weise wird eine Vergiftung des Katalysators und der damit verbundene Aktivitätsverlust verhindert. Im gesamten gesehen kann aber durch die kontinuierliche Zugabe eine große Menge an Ozonolyseprodukten in einem verhältnismäßig kleinem Volumen reduziert werden, wordurch hohe Konzentrationen an Glyoxylsäurealkylesterhalbacetalen entstehen und Zeit sowie Kosten bei der destillativen Entfernung der Lösungsmittel gespart werden.

Als Katalysatoren eignen sich die für Hydrierungen geeigneten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur. Die Ausbeuten sind bei erfindungsgemäßen Verfahren an sich unabhängig von der eingesetzten Katalysatormenge, jedoch empfiehlt es sich zur Erzielung einer ausreichenden Hydriergeschwindigkeit die genannten Katalysatoren in Edelmetallmengen von 0,1 bis 5 Gew.%, vorzugsweise von 0,5 bis 2 Gew.% bezogen auf die jeweils pro Stunde eingespeiste Gesamtmenge der ozonisierten Maleinsäuredialkylester vorzulegen.

Nach Beendigung der Reduktion wird der Katalysator aus dem Reaktionsgemisch abgetrennt und ohne Regenerierung für weitere Hydrierungen verwendet, wobei kein Aktivitätsverlust des Katalysators beobachtet wird.

Beim erfindungsgemäßen Verfahren werden zur Reduktion der Ozonolyseprodukte äquivalente Mengen an Wasserstoff verbraucht. Die Menge an Wasserstoff, die bei der Hydrierung verwendet werden kann, reicht von einem Moläquivalent bis zu einem mehrfachen molaren Überschuß. Die Verwendung von überschüssigem Wasserstoff bringt an sich keine Vorteile und ist nur zweckmäßig, um eine ausreichende Versorgung des Hydrierungsgemisches mit Wasserstoff sicherzustellen.

Die Hydrierung erfolgt beim erfindungsgemäßen Verfahren in vorteilhafter Weise unter praktisch drucklosen Bedingungen. Unter praktisch drucklosen Bedingungen sollen hier Drücke von 1 bis etwa 3 bar verstanden werden, wie das in der Technik üblich ist, um das Eindringen von Luft in den Hydrierreaktor zu verhindern. Auf diese Weise ist die Reduktion der Ozonolyseprodukte technisch sehr einfach durchzuführen. Es ist aber auch möglich, die Hydrierung unter einem Druck bis zu 20 bar durchzuführen und dadurch die Hydriergeschwindigkeit zu steigern.

Die Reduktion verläuft exotherm und wird gemäß einer bevorzugten Ausführungsform der vorliegenden Erfindung bei Temperaturen von 20 bis 40 °C, insbesondere bei Temperaturen im Bereich von 35 bis 40 °C ausgeführt, wobei vorzugsweise ein pH-Wert von 2 bis 4 eingehalten wird.

Da sich im Verlaufe der Hydrierung in geringen Mengen saure Nebenprodukte bilden, erfordert die Einhaltung des pH-Wertes während der Hydrierung eine laufende Einstellung desselben durch dosierte Zugabe einer Base, z. B. NaOH. Die dann in gebundener Form im Hydriergemisch befindliche Base wird zweckmäßig vor der Isolierung des Halbacetals der Formel I, beispielsweise durch Binden mit Mineralsäure, entfernt.

Die nach dem erfindungsgemäßen Verfahren hergestellten Glyoxylsäurealkylesterhalbacetale der Formel I stehen im dynamischen Gleichgewicht mit den entsprechenden Glyoxylsäurealkylesteracetalen und Glyoxylsäurealkylesterhydraten gemäß folgender schematischer Formelübersicht, wobei R und $R_1$ die gleiche Bedeutung wie in Formel I besitzen :

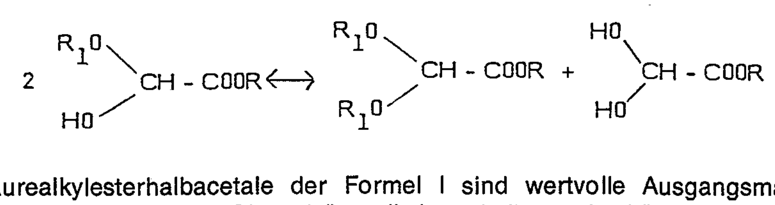

Die Glyoxylsäurealkylesterhalbacetale der Formel I sind wertvolle Ausgangsmaterialien für die Gewinnung von Glyoxylsäure. Die Glyoxylsäurealkylesterhalbacetale können beispielsweise durch Erhitzen in Wasser und abdestillieren des entstandenen Alkohols quantitativ zu Glyoxylsäure verseift

werden. Durch Zugabe von katalytischen Mengen an Säuren oder Zugabe von Basen kann die Verseifung wie auf übliche Weise beschleunigt werden.

Dabei ist es aber nicht notwendig, die Glyoxylsäurealkylesterhalbacetale nach der Hydrierung zu isolieren, sondern die Verseifung kann in kosten- und zeitsparenderweise nach beendeter Hydrierung und Abtrennung des Katalysators direkt in der Hydrierlösung vorgenommen werden.

Das erfindungsgemäße Verfahren wird in den folgenden Beispielen erläutert.

Beispiel 1

In einer Blasensäule werden 900 g (6,25 Mol) Maleinsäuredimethylester in 4 l Methanol vorgelegt. Unter Kühlung auf 0 bis 4 °C wird mit einem Luft/Ozon-Gemisch (3 $m^3$/h Luft, 30 g $O_3/m^3$)3 Stunden und 20 Minuten lang ozonisiert. Dabei wird das Ozon quantitativ aufgenommen, der Restgehalt an Maleinsäuredimethylester beträgt nach beendigter Ozonolyse weniger als 1 % der ursprünglich vorhandenen Maleinsäuredimethylestermenge.

Die bei der Ozonolyse erhaltene Lösung wird portioniert und über ein Dosiergefäß in einen Hydrierreaktor, in dem eine Suspension von 1,5 g Pt in 0,51 Methanol vorgelegt wird und der mit Wasserstoff gefüllt wird, in solchen Dosen eingespeist, daß der Peroxidgehalt im Hydrierreaktor zu Beginn und im Verlaufe der gesamten Hydrierung maximal 0,1 Mol/l beträgt. Unter kräftigem Rühren und Wasserstoffzugabe wird bis zur negativen Peroxidprobe weiterhydriert, wobei über die gesamte Hydrierperiode eine Temperatur von 30 °C-40 °C und durch Zugabe von methanolischer NaOH ein pH-Wert von 2 bis 4 eingehalten wird.

Anschließend wird der Inhalt des Hydrierreaktors bis auf einen Rückstand von 0,5 l über eine Fritte abgesaugt, von neuem ozonierte Lösung über das Dosiergefäß in den Reaktor eingespeist und der Hydriervorgang unter den oben angegebenen Reaktionsbedingungen wiederholt.

Nach Beendigung der Hydrierung wird ein polarographisch bestimmter Glyoxylsäuremethylester-Methanolhalbacetalgehalt von 12,125 mol (97 % der Theorie) festgestellt.

Zur Weiterverarbeitung wird in gebundener Form im Hydrierungsgemisch vorhandene NaOH unter Kühlung vorsichtig mit 98 %iger $H_2SO_4$ als $Na_2SO_4$ ausgefällt und durch Filtration abgetrennt. Das Methanol wird dann am Rotavapor entfernt und der Rückstand bei etwa 55 °C und 25 Torr destilliert. Die Ausbeute an reinem Glyoxylsäuremethylester-Methanolhalbacetal beträgt 1 425 g (11,87 Mol), entsprechend 95 % der Theorie.

Beispiel 2

In einem Reaktor werden 21,6 g (150 mmol) Maleinsäuredimethylester in 100 ml Methanol vorgelgt. Unter Kühlung auf 0 bis 3 °C wird mit einem $O_2/O_3$ Gemisch (60 l $O_2$/h, 1,66 g $O_3$/h) 4 Stunden und 20 Minuten lang ozonisiert. Dabei wird Ozon quantitativ aufgenommen und eine stöchiometrische Menge an Maleinsäuredimethylester verbraucht. Es wird bis zu einem Restgehalt von weniger als 1 % der ursprünglich vorhandenen Maleinsäuredimethylestermenge ozonisiert.

Die bei der Ozonolyse erhaltene Lösung wird über ein Dosiergefäß in einen Hydrierreaktor, in dem eine Suspension von 0,1 g Platin in Methanol vorgelegt wird, unter Rühren und Wasserstoff-Einleitung in solchen Dosen eingespeist, daß der Peroxidgehalt im Hydrierreaktor zu Beginn und im Verlaufe der Hydrierung 0,02 Mol/l nicht übersteigt. Durch äußerliche Kühlung wird das Reaktionsgemisch auf 20 °C gehalten und durch Zugabe von methanolischer NaOH ein pH-Wert von 4 bis 5 eingestellt. Nach Beendigung der Zugabe der Ozonolyselösung wird das Reaktionsgemisch innerhalb von fünf Minuten peroxidfrei. Der Katalysator wird anschließend durch Filtration entfernt und für weitere Hydrierungen verwendet.

Die Gehaltsbestimmung mittels Oximtitration und Polarographie ergibt eine 96 %ige Ausbeute an Glyoxylsäuremethylester-Methanolhalbacetal bezogen auf Maleinsäuredimethylester.

Die Isolierung des Glyoxylsäuremethylester-Methanolhalbacetals erfolgt wie in Beispiel 1 beschrieben.

Beispiel 3

612,5 g (6,25 Mol) Maleinsäureanhydrid werden in 4l Methanol gelöst und unter saurer Katalyse durch Zugabe von einem stark sauren Ionenaustauscher in der H-Form verestert. Ozonolyse und Hydrierung werden, wie in Beispiel 1 angegeben, durchgeführt. Die Ausbeute an Glyoxylsäuremethylester-Methanolhalbacetal beträgt 94 % d. Th.

Beispiel 4

25,83 g Diäthylmaleinat werden in 100 ml Methanol gelöst und ozonisiert. Ozonolyse und Hydrierung werden unter dem in Beispiel 2 angegebenen Bedingungen ausgeführt. Man erhält so 39,1 g (292 mmol) Glyoxylsäureäthylester-Methanolhalbacetal, was einer Ausbeute von 97,3 % d. Th. entspricht.

Beispiel 5

34,25 g Di-n-butylmaleinat werden in 100 ml Methanol gelöst und ozonisiert. Ozonolyse und Hydrierung werden unter den in Beispiel 2 angegebenen Bedingungen ausgeführt. Man erhält so 46,8 g (289 mmol) Glyoxylsäure-n-butylester-Methanolhalbacetal, was einer Ausbeute von 96,3 % d. Th. entspricht.

Beispiel 6

34,25 g (150 mmol) Di-n-butylmaleinat werden mit n-Butanol auf 100 ml verdünnt und mit 150 mmol Ozon wie in Beispiel 2 umgesetzt. Nach der Hydrierung unter den in Beispiel 2 angegebenen Bedingungen erhält man 42,55 g (208,6 mmol) Glyoxylsäure-n-butylester-n-Butanolhalbacetal, was einer Ausbeute von 81,25 % d. Th. entspricht.

Beispiel 7

51,08 g Di-n-oktylmaleinat werden in 100 ml Methanol gelöst und ozonisiert. Ozonolyse und Hydrierung werden unter den in Beispiel 2 angegebenen Bedingungen ausgeführt. Zur Aufarbeitung wird der Katalysator nach beendeter Hydrierung durch Filtration abgetrennt und das Filtrat mit Wasser versetzt und verdünnter $H_2SO_4$ neutralisiert. Dabei scheidet sich das Glyoxylsäure-n-octylester-Methanolhalbacetal als organische Phase ab, die gewaschen, getrocknet und in Vakuum destilliert wird. Man erhält so 61,9 g (284 mmol) Glyoxylsäure-n-octylester-Methanolhalbacetal, was einer Ausbeute von 94,6 % d. Th. entspricht.

Beispiel 8

21,6 g (150 mmol) Dimethylmaleinat werden mit Methanol auf 100 ml aufgefüllt und mit 150 mmol Ozon wie in Beispiel 2 zu Reaktion gebracht. Die bei der Ozonolyse erhaltene Lösung wird über ein Dosiergefäß in einen Hydrierreaktor, in dem eine Suspension eines 10 %igen Pd-Katalysators auf Kohle vorgelegt wird, eingespeist und unter den in Beispiel 2 angegebenen Bedingungen hydriert. Der Anssatz ist nach weniger als 10 Minuten peroxidfrei.

Die polarographische Gehaltsbestimmung ergibt einen Gehalt von 243,8 mmol Glyoxylsäuremethylester-Methanolhalbacetal, was einer Ausbeute von 81,25 % d. Th. entspricht.

Beispiel 9

85 g (0,7 Mol) eines 98,5 %igen Glyoxylsäuremethylester-Methanolhalbacetals werden mit 100 $H_2O$ so lange erhitzt, bis 55 g Methanol/Wasser-Destillat bei einer Sumpftemperatur von 105 °C übergegangen sind. Es verbleiben 130 g wässerige Glyoxylsäurelösung mit einem Gehalt von 0,69 Mol Glyoxylsäure.

In analoger Weise können auch die Halbacetale gemäß den Beispielen 4, 5, 6 und 7 zu Glyoxylsäure verseift werden.

**Patentansprüche**

1. Verfahren zur Herstellung von Glyoxylsäurealkylesterhalbacetalen der allgemeinen Formel

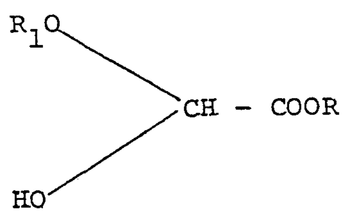

(I)

in der R einen Alkylrest mit 1 bis 10 C-Atomen und $R_1$ einen Alkylrest mit 1 bis 4 C-Atomen bedeuten, durch Ozonolyse eines Maleinsäurederivats gefolgt von katalytischer Hydrierung der Ozonolyseprodukte, dadurch gekennzeichnet, daß man

a) einen Maleinsäuredialkylester mit 1 bis 10 C-Atomen im Alkylrest in einem aliphatischen Alkohol der Formel $R_1OH$, wobei $R_1$ die oben angegebene Bedeutung hat, löst und bei Temperaturen von − 80 bis + 20 °C mit der äquivalenten Menge Ozon umsetzt, worauf man

b) die dabei entstehende peroxidhaltige Lösung in eine Suspension des Hydrierkatalysators in dem in Stufe a) bei der Ozonolyse verwendeten Alkohol der Formel $R_1OH$ mit $R_1$ mit der oben angegebenen Bedeutung kontinuierlich in einer solchen Dosierung einspeist, daß über den ganzen

Verlauf der Hydrierung im Hydrierreaktor ein Peroxidgehalt von maximal 0,1 Mol/l eingestellt und aufrecht erhalten wird, und bei pH 2 bis pH 7 bei Temperaturen von 15 °C bis 45 °C unter einem Druck von 1 bis 20 bar hydriert.

2. Verfahren nach Anspruch 1, dadurch gekennzeichnet, daß man die Ozonolyse in Stufe a) bei Temperaturen im Bereich von − 10 bis + 5 °C durchführt.

3. Verfahren nach den Ansprüchen 1 und 2, dadurch gekennzeichnet, daß man als Alkohol der Formel $R_1OH$ Methanol verwendet.

4. Verfahren nach den Ansprüchen 1 bis 3, dadurch gekennzeichnet, daß während der gesamten Hydrierung in Stufe b) im Hydrierreaktor ein Peroxidgehalt von höchstens 0,02 Mol/l eingestellt und aufrecht erhalten wird.

5. Verfahren nach den Ansprüchen 1 bis 4, dadurch gekennzeichnet, daß bei der Hydrierung in Stufe b) ein trägerfreier Platinkatalysator verwendet wird.

6. Verfahren nach den Ansprüchen 1 bis 5, dadurch gekennzeichnet, daß während der Hydrierung in Stufe b) eine Temperatur von 35 bis 40 °C aufrecht erhalten wird.

7. Verfahren nach den Ansprüchen 1 bis 6, dadurch gekennzeichnet, daß während der Hydrierung in Stufe b) ein pH-Wert von 2 bis 4 eingestellt wird.

8. Verfahren zur Herstellung von Glyoxylsäure, dadurch gekennzeichnet, daß ein Glyoxylsäurealkylesterhalbacetal der allgemeinen Formel I, welches nach den Ansprüchen 1-7 hergestellt wurde, verseift wird.

9. Verfahren nach Anspruch 8, dadurch gekennzeichnet, daß das Glyoxylsäurealkylesterhalbacetal der Formel I durch Erhitzen mit Wasser und Abdestillieren des Alkohol/Wasser-Gemisches verseift wird.

10. Verfahren nach den Ansprüchen 8 und 9, dadurch gekennzeichnet, daß das Glyoxylsäurealkylesterhalbacetal der Formel I unmittelbar nach der Hydrierung und dem Abtrennen des Katalysators ohne Isolierung des Halbacetals der Formel I verseift wird.

## Claims

1. Process for the preparation of alkyl glyoxylate semi-acetals of the general formula

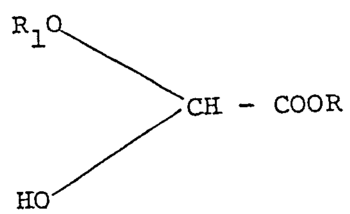

(I)

in which R denotes an alkyl radical with 1 to 10 C atoms and $R_1$ an alkyl radical with 1 to 4 C atoms, by ozonolysis of a maleic acid derivative followed by catalytic hydrogenation of the ozonolysis products, characterised in that

a) a dialkyl maleate with 1 to 10 C atoms in the alkyl radical is dissolved in an aliphatic alcohol of the formula $R_1OH$, wherein $R_1$ has the abovementioned meaning, and is reacted at temperatures of − 80 to + 20 °C with an equivalent amount of ozone, after which

b) the resulting peroxide-containing solution is fed continuously into a suspension of the hydrogenation catalyst in the alcohol of the formula $R_1OH$, used in the ozonolysis in stage a), $R_1$ having the abovementioned meaning, at a rate of addition such that over the entire course of the hydrogenation a peroxide content of at most 0.1 mole/l is set up, and maintained, in the hydrogenation reactor, the hydrogenation being carried out at pH 2 to pH 7 at temperatures of 15 °C to 45 °C under a pressure of 1 to 20 bar.

2. Process according to Claim 1, characterised in that the ozonolysis in stage a) is carried out at temperatures in the range from − 10 to + 5 °C.

3. Process according to Claims 1 and 2, characterised in that methanol is used as the alcohol of the formula $R_1OH$.

4. Process according to Claims 1 to 3, characterised in that over the entire hydrogenation in stage b) a peroxide content of a most 0.02 mole/l is set up and maintained in the hydrogenation reactor.

5. Process according to Claims 1 to 4, characterised in that an unsupported platinum catalyst is used in the hydrogenation in stage b).

6. Process according to Claims 1 to 5, characterised in that a temperature of 35 to 40 °C is maintained during the hydrogenation in stage b).

7. Process according to Claims 1 to 6, characterised in that a pH value of 2 to 4 is set up during the hydrogenation in stage b).

8. Process for the preparation of glyxoylic acid, characterised in that an alkyl glyoxylate semi-acetal of the general formula I, which has been prepared according to Claims 1-7, is hydrolysed.

9. Process according to Claim 8, characterised in that the alkyl glyxoylate semi-acetal of the formula I is hydrolysed by heating with water and distilling off the alcohol/water mixture.

10. Process according to Claims 8 and 9, characterised in that the alkyl glyoxylate semi-acetal of the formula I is hydrolysed immediately after the hydrogenation and after removal of the catalyst, but without isolation of the semi-acetal of the formula I.

**Revendications**

1. Procédé pour la préparation d'hémi-acétals d'esters alcoyliques d'acide glyoxylique de formule générale I :

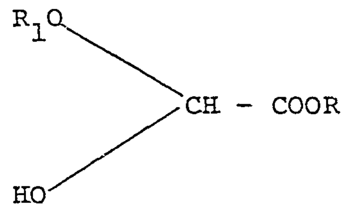

(I)

dans laquelle R est un radical alcoyle ayant de 1 à 10 atomes de C et $R_1$ est un radical alcoyle ayant de 1 à 4 atomes de C, par ozonolyse d'un dérivé d'acide maléique, suivie d'une hydrogénation catalytique des produits d'ozonolyse, caractérisé en ce que :

a) on fait dissoudre un ester dialcoylique d'acide maléique de 1 à 10 atomes de C dans le radical alcoyle dans un alcool aliphatique de formule $R_1OH$, dans laquelle $R_1$ a la signification indiquée précédemment, et on fait réagir à des températures de − 80 à + 20 °C avec la quantité équivalente d'ozone, après quoi

b) on introduit la solution à teneur en peroxyde alors formée dans une suspension du catalyseur d'hydrogénation dans l'alcool de formule $R_1OH$ utilisé au stade a) lors de l'ozonolyse, formule dans laquelle $R_1$ a la signification indiquée, en opérant de façon continue selon un dosage tel que pendant toute l'évolution de l'hydrogénation, on règle et on maintienne dans le réacteur d'hydrogénation une teneur en peroxyde de 0,1 mole/l au maximum, et on effectue l'hydrogénation à un pH de 2 à 7 à des températures de 15 °C à 45 °C sous une pression de 1 à 20 bars.

2. Procédé suivant la revendication 1, caractérisé en ce qu'on effectue l'ozonolyse au cours du stade a) à des températures comprises dans une gamme allant de − 10 à + 5 °C.

3. Procédé suivant les revendications 1 et 2, caractérisé en ce qu'on utilise comme alcool de formule $R_1OH$ du méthanol.

4. Procédé suivant l'une quelconque des revendications 1 à 3, caractérisé en ce que, pendant la totalité de l'hydrogénation au cours du stade b), on règle et on entretient dans le réacteur d'hydrogénation une teneur en peroxyde de 0,02 mole/l au maximum.

5. Procédé suivant l'une quelconque des revendications 1 à 4, caractérisé en ce qu'on utilise lors de l'hydrogénation au cours du stade b) un catalyseur au platine sans support.

6. Procédé suivant l'une quelconque des revendications 1 à 5, caractérisé en ce qu'on maintient pendant l'hydrogénation au cours du stade b) une température de 35 à 40 °C.

7. Procédé suivant l'une quelconque des revendications 1 à 6, caractérisé en ce qu'on règle pendant l'hydrogénation au cours du stade b) une valeur de pH de 2 à 4.

8. Procédé pour la préparation d'acide glyoxylique caractérisé en ce qu'on saponifie un hémi-acétal d'ester alcoylique d'acide glyoxylique de formule générale I, qui a été préparé suivant l'une quelconque des revendications 1 à 7.

9. Procédé suivant la revendication 8, caractérisé en ce qu'on saponifie l'hémi-acétal d'ester alcoylique d'acide glyoxylique de formule I par chauffage avec de l'eau élimination par distillation du mélange alcool/eau.

10. Procédé suivant l'une quelconque des revendications 8 et 9, caractérisé en ce qu'on saponifie l'hémi-acétal d'ester alcoylique d'acide glyoxylique de formule I directement après l'hydrogénation et après séparation du catalyseur, sans isolement de l'hémi-acétal de formule I.